(19) Europäisches Patentamt
European Patent Office
Office européen des brevets

(11) Numéro de publication: **0 265 467**
**B1**

(12) **FASCICULE DE BREVET EUROPEEN**

(45) Date de publication du fascicule du brevet: **04.07.90**

(51) Int. Cl.$^5$: **B 01 J 13/02**, A 61 K 9/50, A 61 K 7/00

(21) Numéro de dépôt: **87902547.6**

(22) Date de dépôt: **17.04.87**

(86) Numéro de dépôt international: **PCT/FR87/00127**

(87) Numéro de publication internationale: **WO 87/06499 05.11.87 Gazette 87/24**

(54) **PROCEDE POUR FACILITER LA FORMATION DE SPHERULES LIPIDIQUES EN DISPERSION DANS UNE PHASE AQUEUSE ET POUR AMELIORER LEUR STABILITE ET LEUR TAUX D'ENCAPSULATION, ET DISPERSIONS CORRESPONDANTES.**

(30) Priorité: **22.04.86 FR 8605775**

(43) Date de publication de la demande: **04.05.88 Bulletin 88/18**

(45) Mention de la délivrance du brevet: **04.07.90 Bulletin 90/27**

(84) Etats contractants désignés: **BE IT**

(56) Documents cités:
**EP-A-0 102 324** **EP-A-0 170 247**
**EP-A-0 119 020** **EP-A-0 172 007**
**EP-A-0 160 286** **US-A-4 544 545**

**Biochemie, 63, p.795-798 (1981)**

**Le dossier contient des informations techniques présentées postérieurement au dépôt de la demande et ne figurant pas dans le présent fascicule.**

(73) Titulaire: **L'OREAL**
**14, Rue Royale**
**F-75008 Paris (FR)**

(72) Inventeur: **HANDJANI, Rose-Marie**
**17 bis rue Campagne-Première**
**F-75014 Paris (FR)**
Inventeur: **RIBIER, Alain**
**2, Boulevard Jourdan**
**F-75014 Paris (FR)**
Inventeur: **VANLERBERGHE, Guy**
**Rue du Général de Gaulle Montjay la Tour**
**F-77410 Villevaude (FR)**

(74) Mandataire: **Peuscet, Jacques et al**
**Cabinet Peuscet 68, rue d'Hauteville**
**F-75010 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 265 467 B1**

## Description

La présente invention concerne les dispersions aqueuses de sphérules lipidiques, ces dispersions étant utilisables dans le domaine cosmétique, pharmaceutique et alimentaire. Elle a plus précisément pour objet un procédé pour faciliter la formation de ces sphérules lipidiques et pour améliorer simultanément leur stabilité dans les dispersions aqueuses, ainsi que leur taux d'encapsulation.

On sait que certains lipides possèdent la propriété de former, en présence d'eau, des phases mésomorphes dont l'état d'organisation est intermédiaire entre l'état cristallin et l'état liquide. Parmi les lipides qui donnent naissance à des phases mésomorphes, il a déjà été indiqué que certains peuvent gonfler en solution aqueuse pour former des sphérules dispersées dans le milieu aqueux, ces sphérules étant constituées par des couches multi-moléculaires et de préférence des couches bi-moléculaires ayant une épaisseur approximative de 30 à 100 Å (voir, en particulier, l'article de Bangham, Standish et Watkins, J. Mol. Biol., 13, 238 (1965)).

Dans le brevet français n° 2 315 991, on a déjà décrit des dispersions de sphérules lipidiques; ces sphérules sont caractérisées par leur structure en feuillets constituée de deux ou plusieurs couches lipidiques séparées les unes des autres par des couches de phase aqueuse; elles peuvent ainsi servir à encapsuler, dans les compartiments aqueux compris entre les couches lipidiques, des substances actives hydrosolubles, par exemple pharmaceutiques ou cosmétiques, et à les protéger des conditions extérieures. Les composés lipidiques utilisables pour constituer de telles sphérules peuvent être des composés ioniques, auquel cas on obtient des liposomes, ou des composés non-ioniques, auquel cas on obtient des niosomes.

Dans les brevets français n° 2 485 921 et 2 490 504, on a également décrit des compositions consistant en une dispersion aqueuse de sphérules du type précité dans la phase aqueuse externe desquelles on prévoyait une dispersion d'huile. On a constaté que, de façon surprenante, la présence de sphérules lipidiques permettait de stabiliser la dispersion d'huile et qu'en outre, on obtenait, avec de telles compositions, un effet combiné des sphérules et des gouttelettes d'huile, ce qui présentait un avantage important dans le domaine de la cosmétique ou de la pharmacie.

Dans le brevet français n° 2 543 018, on a proposé, en outre, un procédé de préparation de vésicules lipidiques unilamellaires ayant un diamètre moyen supérieur à 1000 Å.

Lorsque l'on prépare des liposomes ou des niosomes, divers additifs peuvent être associés aux composés lipidiques ioniques ou non-ioniques, en vue de modifier la perméabilité ou la charge superficielle des sphérules. On a cité, à cet égard, un certain nombre de ces additifs dans les brevets français précités.

On sait ainsi que, lorsque l'on veut diminuer la perméabilité des vésicules, on peut ajouter, aux composés lipidiques, des stérols, principalement du chloestérol, ces stérols augmentant la rigidité des multi-couches.

On sait également que l'incorporation de molécules à charges électriques dans les parois des vésicules liposomes ou niosomes, affecte les propriétés de ces multi-couches. Le rôle des lipides chargés, tels que le dicétylphosphate, l'acide phosphatidique, les amines ou les ammonium quaternaires à longues chaînes hydrocarbonées, est d'améliorer la stabilité des vésicules en prévenant leur floculation et, de ce fait, leur fusion, même en présence d'électrolytes, et de permettre l'augmentation du taux d'encapsulation de substances hydrosolubles en accroissant l'épaisseur des feuillets aqueux qui séparent les multi-couches lipidiques.

Il a, en outre, été mis en évidence, par A. Colombat et al., Biochimie (1981), *63*, 795—798, que le cholestérol phosphate, c'est-à-dire, un ester hydrophile du cholestérol, combine les effets d'un amphiphile chargé, à savoir d'augmenter la stabilité des sphérules et leur taux d'encapsulation, et l'effet du cholestérol, à savoir de diminuer la perméabilité des sphérules.

On observe cependant que l'introduction de plus de 5% en poids de lipides chargés dans la membrane vésiculaire entraîne, soit une perméabilité forte aux solutés, soit une recristallisation du lipide chargé.

Cependant, la Société Déposante a découvert que, d'une façon suprenante, un lipide chargé particulier, à savoir le cholestérol-sulfate, ne présentait pas ces inconvénients et se distinguait des autres lipides chargés par le fait que l'on peut en introduire jusqu'à 50% en poids dans la membrane lipidique, sans constater de recristallisation, et qu'à des pourcentages déjà élevés dans la membrane (25% en poids) il n'induit qu'une faible perméabilité.

Ceci constitue une observation très intéressante, d'autant plus qu'au cours de ces dernières années, le cholestérol-sulfate—qui, contrairement au cholestérol phosphate est présent dans le revêtement cutané—a été reconnu comme jouant un rôle capital dans la cohésion cellulaire (travaux d'Epstein, Williams, Elias). Il en résulte que l'on peut espérer que, pour des applications cutanées, les vésicules contenant du cholestérol-sulfate vont présenter une très grande efficacité.

La présente invention a donc pour objet un procédé pour faciliter la formation de sphérules lipidiques en dispersion dans un milieu aqueux D, et pour améliorer simultanément la stabilité et le taux d'encapsulation desdites sphérules lipidiques, celles-ci étant constituées de feuillets lipidiques sensiblement concentriques encapsulant une phase aqueuse E, le(s) lipide(s) constitutif(s) des feuillets étant des amphiphiles ioniques ou non-ioniques, caractérisé par le fait qu'avant la formation desites sphérules, on ajoute au(x) lipide(s), destiné(s) à constituer les feuillets des sphérules, au moins un

cholestérol-sulfate de cation ammonium, alcalin ou alcalino-terreux, à raison de 1 à 50% en poids par rapport au poids total de la phase lipidique.

En pratique, le seuil maximal est fonction de la nature du lipide utilisé et il peut se situer entre 20 et 50% en poids dans la phase lipidique des sphérules.

On utilise, de préférence, un cholestérol-sulfate d'ammonium, substitué ou non, de sodium ou de potassium.

L'invention a également pour objet une dispersion de sphérules lipidiques obtenue par le procédé ci-dessus défini, ledit procédé incorporant éventuellement les caractéristiques complémentaires qui vont être détaillées ci-après.

Pour réaliser la dispersion dans la phase aqueuse D des sphérules lipidiques, on peut utiliser n'importe lequel des procédés antérieurement connus et décrits.

On peut, par exemple, utiliser le procédé, qui consiste à dissoudre les lipides dans un solvant volatil, à former un film mince de lipides sur les parois d'un flacon par évaporation du solvant, à introduire dans ledit flacon la phase aqueuse E à encapsuler et à agiter le mélange mécaniquement jusqu'à l'obtention de la dispersion de sphérules à la taille désirée; dans ce cas, les phases aqueuses D et E sont nécessairement identiques.

On peut aussi utiliser le procédé décrit dans le brevet français n° 2 315 991, qui consiste à former une phase lamellaire plane par introduction de la phase aqueuse à encapsuler E dans les lipides liquides, à une température légèrement supérieure à la température de fusion des lipides, à ajouter ensuite à la phase lamellaire obtenue une phase aqueuse de dispersion D, qui peut être identique ou non à la phase aqueuse E, et à agiter énergiquement, par exemple mécaniquement, pour obtenir le passage de la phase lamellaire plane à une dispersion, dans la phase aqueuse D, de sphérules lipidiques encapsulant la phase aqueuse E. Selon les moyens utilisés pour réaliser la dispersion (ultra-disperseur, homogénéiseur, et/ou ultrasons) et selon le temps d'agitation (de 15 minutes à quelques heures), on obtient des sphérules, dont le diamètre moyen varie de 0,025 à 5 microns environ.

Le procédé sus-indiqué convient particulièrement bien, lorsque l'on souhaite utiliser des sphérules multi-lamellaires. Dans le cas où l'on désire des sphérules unilamellaires, on peut utiliser, pour leur préparation, le procédé décrit dans le brevet français n° 2 543 018; selon ce procédé, on solubilise les lipides destinés à former le feuillet des vésicules dans au moins un solvant insoluble dans l'eau; on conditionne la solution lipidique à l'état liquide, dans un récipient, à une pression $P_1$ et à une température $\theta_1$; on conditionne la phase aqueuse à encapsuler E à une pression $P_2$ et à une température $\theta_2$, et on injecte la solution lipidique dans la phase aqueuse de telle sorte que le(s) solvant(s) de la solution lipidique se vaporise(nt) lorsqu'il(s) arrive(nt) au contact de ladite phase aqueuse, ladite injection étant réalisée avec un débit réduit pour constituer initialement des gouttelettes, la pression $P_2$ étant inférieure à la pression $P_1$ et à la tension de vapeur du (ou des) solvant(s) dans lesdites gouttelettes à la température $\theta_2$.

Comme indiqué ci-dessus, le cholestérol-sulfate est ajouté à n'importe quel moment avant la formation des vésicules, c'est-à-dire lorsque l'on passe par la formation d'une phase lamellaire, soit avant la préparation de ladite phase lamellaire, soit après.

Les lipides utilisés pour la préparation des sphérules sont des amphiphiles d'origine naturelle ou synthétique, ioniques ou non-ioniques, comportant, par molécule, une ou plusieurs longue(s) chaîne(s) hydrocarbonée(s), saturée(s) ou insaturée(s), linéaire(s) ou ramifiée(s), ayant notamment de 8 à 30 atomes de carbone, comme les chaînes oléique, lanolique, tétradécylique, hexadécylique, isostéarylique, laurique ou alcoyphényle, et un ou plusieurs groupement(s) hydrophile(s) pris parmi les groupes hydroxyle, éthéroxyde, carboxyle, phosphate et amine.

Parmi les amphiphiles ioniques, on préfère utiliser les phospholipides naturels (par exemple la lécithine d'oeuf ou de soja ou la sphingomyéline), les phospholipides de synthèse (par exemple, la dipalmitoyl-phosphatidylcholine ou la lécithine hydrogénée); on peut aussi utiliser les composés amphotères comportant deux chaînes lipophiles ou une association de deux ions organiques à longue chaîne de signes opposés ainsi que les composés anioniques.

Parmi les composés anioniques, on citera ceux qui sont décrits dans la demande de brevet luxembourgeois n° 85 971 déposée le 23 Juin 1985 et qui sont représentés par la formule:

$$\begin{array}{l} R_1\text{—CHOH—CH—COOM} \\ \qquad\qquad\qquad\ | \\ \qquad\quad R_2\text{—CONH} \end{array}$$

formule dans laquelle:

— $R_1$ désigne un radical alcoyle ou alcényle en $C_7$—$C_{21}$;

— $R_2$ désigne un radical hydrocarboné, saturé ou insaturé, en $C_7$—$C_{31}$; et

— M représente H, Na, K, $NH_4$ ou un ion ammonium substitué dérivé d'une amine.

Les composés anioniques définis au précédent paragraphe peuvent être obtenus par le procédé de préparation mentionné dans la demande de brevet français 2 588 256.

Pour les amphiphiles non-ioniques, on préfère que les groupes hydrophiles soient des groupes polyoxyéthylénés ou polyglycéroles, ou des groupes dérivant d'esters de polyols oxyéthylénés ou non ou

encore des dérivés d'hydroxyamides. Avantageusement, ces composés lipidiques non-ioniques sont choisis dans le groupe formé par

— les éthers de polyglycérol, linéaires ou ramifiés, de formules respectives:

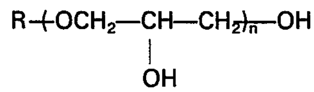

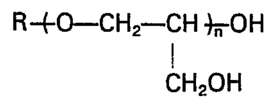

$\bar{n}$ étant une valeur statistique moyenne comprise entre 1 et 6, R étant une chaîne aliphatique linéaire ou ramifiée, saturée ou insaturée, comprenant de 12 à 30 atomes de carbone, les radicaux hydrocarbonés des alcools de lanoline ou restes hydroxy-2 alkyle des α-diols à longue chaîne;

— les éthers de polyglycérol, linéaires ou ramifiés, comportant deux chaînes grasses;

— les alcools gras polyoxyéthylénés;

— les stérols polyoxyéthylénés;

— les éthers de polyols;

— les esters de polyols oxyéthylénés ou non et, en particulier, les esters de sorbitol polyoxyéthylénés;

— les glycolipides d'origine naturelle ou synthétique, par exemple les cérébrosides;

— les hydroxyamides tels que ceux qui sont décrits dans la demande de brevet luxembourgeois n° 85 971 déposée le 23 Juin 1985 et qui sont représentés par la formule:

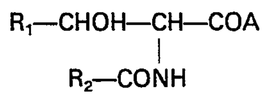

formule dans laquelle:

— $R_1$ désigne un radical alcoyle ou alcényle en $C_7$—$C_{21}$;

— $R_2$ désigne un radical hydrocarboné, saturé ou insaturé, en $C_7$—$C_{31}$;

— COA désigne un groupement choisi parmi les deux groupements suivants:

— un reste

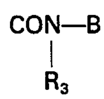

B étant un radical dérivé d'amines primaires ou secondaires, mono- ou polyhydroxylés et $R_3$ désignant un atome d'hydrogène ou un radical méthyle, éthyle ou hydroxyéthyle;

— COOZ, Z représentant le reste d'un polyol en $C_3$—$C_7$.

De façon connue, divers additifs peuvent être associés aux composés lipidiques en vue de modifier la perméabilité ou la charge superficielle des sphérules. On citera, à cet égard, l'addition éventuelle des alcools et diols à longue chaîne, des stérols, par exemple le cholestérol et le β-sitostérol, des amines à longue chaîne, des hydroxyalkylamines, des amines grasses polyoxyéthylénées, des esters d'amino-alcools à longue chaîne, de leurs sels, des esters phosphoriques d'alcools gras, par exemple le dicétylphosphate de sodium et des alkylsulfates, par exemple le cétylsulfate de sodium, des dérivés ioniques des stérols autres que les cholestérolsulfates.

Avantageusement, on peut utiliser, pour constituer la dispersion de sphérules, de 0,5 à 25% en poids d'amphiphile(s) par rapport au poids total de la dispersion de sphérules à obtenir.

On peut prévoir que les parois des sphérules renferment au moins une substance liposoluble active telle que, par exemple, un agent kératolitique comme l'acide rétinoïque, un agent anti-inflammatoire, comme le 17-valérate de β-méthasone, un agent anti-oxydant, comme la vitamine E et son acétate ou le palmitate d'ascorbyle, ce qui est particulièrement intéressant lorsqu'on envisage des applications topiques.

On peut aussi prévoir que la phase aqueuse E à encapsuler dans les sphérules soit une solution aqueuse de substance(s) active(s), de préférence isoosmotique(s) par rapport à la phase D de la dispersion. Les phases D et E peuvent être identiques.

Pour une composition cosmétique, la phase aqueuse E encapsulée dans les sphérules peut contenir, par exemple, au moins un produit pris dans le groupe formé par les humectants, tels que la glycérine, le sorbitol, le pentaérythritol, l'inositol, l'acide pyrrolidonecarboxylique et ses sels; les agents de brunissage artificiel, tels que la dihydroxyacétone, l'érythrulose, le glycéraldéhyde, les γ-dialdéhydes, tels que l'aldéhyde tartrique, éventuellement associés à d'autres agents de coloration de la peau; les agents anti-solaires hydrosolubles; les anti-perspirants; les déodorants; les astringents; les produits rafraîchissants, toniques, cicatrisants, kératolytiques, dépilatoires; les extraits de tissus d'animaux ou végétaux; les eaux

parfumées; les colorants hydrosolubles; les agents antipelliculaires; les agents anti-séborrhéiques; les oxydants tels que l'eau oxygénée et les réducteurs tels que l'acide thioglycolique et ses sels.

Dans le cas d'une composition utilisable en pharmacie, la phase aqueuse E encapsulée dans les sphérules contient, de préférence, au moins un produit pris dans le groupe formé par les vitamines, les hormones, les enzymes, tels que la superoxyde dismutase, les vaccins, les anti-inflammatoires, tels que l'hydrocortisone, les antibiotiques et les bactéricides.

On peut également prévoir que la phase aqueuse D entourant les sphérules contienne au moins une phase liquide non miscible à l'eau dispersée dans ladite phase aqueuse D. Cette phase liquide non miscible à l'eau peut être une huile ou un constituant pris dans le groupe formé par les hydrocarbures, les carbures halogénés, les polysiloxanes, les esters d'acides organiques, les éthers et polyéthers. Avantageusement, la quantité de phase liquide non miscible à l'eau, dispersée dans la phase aqueuse D, est comprise entre 2 et 70% en poids par rapport au poids total de la composition, la proportion pondérale relative de lipide(s) amphiphile(s) constitutif(s) de sphérules par rapport à la (ou aux) phase(s) liquide(s) non miscible(s) à l'eau, dispersée(s), étant comprise entre 0,02/1 et 10/1.

L'huile utilisée pour être dispersée dans la phase aqueuse D est avantageusement prise dans le groupe formé par les esters d'acides gras et de polyols, notamment les triglycérides liquides, et les esters d'acides gras et d'alcools ramifiés de formule $R_4$—$COOR_5$, formule dans laquelle $R_4$ représente le reste d'un acide gras supérieur comportant de 7 à 19 atomes de carbone et $R_5$ représente une chaîne hydrocarbonée ramifiée contenant de 3 à 20 atomes de carbone. Dans un tel cas, si l'huile est un ester d'acides gras et de polyols, on préfère qu'elle soit choisie dans le groupe formé par les huiles de tournesol, de maïs, de soja, de courge, de pépins de raisins, de jojoba, de sésame et le tri-caprocaprylate de glycérol; si, au contraire, l'huile est un ester d'acides gras supérieur et d'alcool ramifié, on préfère que ladite huile soit l'huile de Purcellin.

Pour constituer la phase liquide non miscible à l'eau, on peut encore avantageusement choisir l'hexadécane, l'huile de paraffine, le perhydrosqualène, la perfluorotributylamine, le perfluorodécahydronaphtalène et l'huile de silicone volatile.

On peut également prévoir que la phase aqueuse D, qui entoure les sphérules, contienne au moins un adjuvant pris dans le groupe formé par les opacifiants, les gélifiants, les arômes, les parfums, les filtres anti-solaires et les colorants, ceux de ces adjuvants qui sont liposolubles pouvant être dissous dans la phase liquide non miscible à l'eau dispersée dans la phase aqueuse D, au cas où on utilise une telle dispersion.

Si le liquide non miscible à l'eau dispersé rajouté dans la phase aqueuse continue qui entoure les sphérules, doit contenir des adjuvants dissous, la dissolution de ces adjuvants est réalisée avant d'effectuer la dispersion.

De tels adjuvants peuvent être par exemple des filtres anti-solaires, tels que le paradiméthylamino benzoate de 2-éthyl hexyle ou des substances destinées à améliorer l'état des peaux sèches ou séniles, en particulier des insaponifiables tels que des insaponifiables de soja, d'avocat, des tocophérols, des vitamines E, F, des anti-oxydants.

La dispersion d'huile dans l'eau qui constitue le milieu externe de la dispersion de sphérules peut contenir au moins un additif, notamment un gélifiant ou un parfum. L'additif est ajouté à la dispersion en même temps que l'huile. Le gélifiant peut être introduit à une concentration variant entre 0,1 et 2%, ces pourcentages étant exprimés en poids par rapport au poids total de la composition. Parmi les gélifiants utilisables, on peut citer les dérivés de cellulose les dérivés d'algues; les gommes naturelles ou encore les acides polyacryliques réticulés. On préfère utiliser, à titre de gélifiants, l'hydroxyéthyl cellulose, l'acide polyacrylique réticulé vendu par la société GOODRICH sous la dénomination commerciale "CARBOPOL® 940", le satiagum, l'adragante ou encore les polyglucoses.

Quand on réalise une composition comportant une dispersion de liquide(s) non miscible(s) à l'eau, on constate que cette dispersion est stable sans utilisation d'émulsionnant.

Si la dispersion de sphérules comporte des sphérules de plusieurs types, par exemple des niosomes et des liposomes, on prépare séparément les deux types de sphérules et on mélange les deux dispersions.

Pour mieux illustrer l'objet de la présente invention, on va indiquer maintenant des résultats d'essais comparatifs démontrant que l'introduction de cholestérol sulfate dans la phase lipidique de sphérules en dispersion dans l'eau conduit, pour ces sphérules, à une perméabilité nettement améliorée, et à un taux d'encapsulation qui est toujours au moins égal à celui obtenu sans l'introduction de cholestérol-sulfate ou avec l'introduction d'un lipide chargé autre que le cholestérolsulfate.

Ces essais comparatifs sont résumés dans le tableau ci-après.

TABLEAU

| Type de phase lipidique | Composition de la phase lipidique | Gonflement au glucose en µl/mg | Perméabilité (%) après (n) jours |
|---|---|---|---|
| Non-ionique | A 47,5% X=DCP<br>Ch 47,5%<br>X 5% X=ChS | 13<br><br>13 | 5 (30)<br><br>0 (30) |
| Anionique | B 60%<br>Ch 40% | 15 | 63 (30) |
| | B 60%<br>Ch 35%<br>ChS 5% | 15 | 39 (30) |
| Amphotère | C 75%<br>Ch 20% X=DCP<br>X 5% X=ChS | 3<br>9 | 64 (o) 80 (l)<br>16 (o) 65 (l) |

Dans ce tableau, les abréviations A, B, C, Ch, DCP et ChS ont les significations respectives suivantes:

A: lipide non-ionique représenté par la formule suivante:

$$R{-}O{-}\left(CH_2{-}\underset{\displaystyle CH_2OH}{\underset{|}{CH}}{-}O\right)_{\bar{n}}{-}H$$

dans laquelle $R=C_{16}H_{33}$ et $\bar{n}$ est une valeur statistique moyenne égale à 3.

B: lipide anionique dont la préparation est décrite à l'exemple 5 de la demande de brevet luxembourgeois n° 85 971 et qui est représenté par la formule suivante:

$$R_1{-}CHOH{-}\underset{\displaystyle R_2{-}CONH}{\underset{|}{CH}}{-}COOH,NH_2CH_2CHOH{-}CH_3$$

dans laquelle $R_1=C_{15}H_{31}$ et $R_2$ désigne un mélange de chaînes hydrocarbonées de formules:

$(CH_2)_7CH=CH{-}(CH_2)_7CH_3$

$(CH_2)_{14}CH_3$

$(CH_2)_{12}CH_3$

C: lécithine de soja (phosphatidyl choline)

Ch: cholestérol

DCP: dicétylphosphate (forme acide)

ChS: cholestérol-sulfate de sodium

L'expérience, qui est rapportée dans le cas de lipides non-ioniques, démontre que la perméabilité après trente jours est de 0% lors de l'introduction de cholestérol-sulfate, alors qu'elle était de 5% lors de l'introduction du dicétylphosphate, au même taux de 5% en poids par rapport à la phase lipidique.

Il en est de même pour l'expérience qui concerne les lipides anioniques, la perméabilité étant diminuée, lorsque l'on remplace le cholestérol par un mélange (cholestérol/cholestérol-sulfate) dans la composition lipidique, le taux d'encapsulation restant le même.

Enfin, l'expérience comparative rapportée dans le cas de l'utilisation de lipides amphotères pour constituer les sphérules, montre que le remplacement du dicétylphosphate par le cholestérol-sulfate améliore le taux d'encapsulation, de même d'ailleurs que la perméabilité.

On va donner ci-après quelques exemples de préparation mettant en oeuvre l'invention et quelques exemples de formulation illustrant l'utilisation des dispersions de sphérules selon l'invention.

La préparation des formules cosmétiques ou pharmaceutiques données dans les exemples ci-dessous s'effectue en une ou deux phases.

Dans la première phase, on fabrique une dispersion aqueuse selon le procédé décrit dans le brevet français 2 315 991 (exemples 1 à 3). La dispersion aqueuse de sphérules lipidiques est préparée à partir:

— d'un lipide amphiphile non-ionique ou anionique ou amphotère,

— du cholestérol-sulfate, utilisé seul ou associé au cholestérol,

— de substances cosmétiques actives de nature liposoluble et/ou hydrosoluble et d'eau déminéralisée.

Dans une deuxième phase, facultative, selon le caractère cosmétique ou pharmaceutique de la

formulation, on pourrait ajouter de l'huile dans le milieu externe selon les critères et procédés décrits dans les brevets français 2 485 921 et 2 532 191.

Pour les compositions cosmétiques, on pourra également ajouter différentes substances cosmétiques, telles que parfum ou gélifiants, par exemple.

Exemple 1:—Creme de soins pour peaux seches

1ère phase de préparation:

Dans un bécher en acier inoxydable, on pèse les produits suivants:

— lipide amphiphile non-ionique de formule

$$R-\!\!\left(OCH_2-\underset{\displaystyle CH_2OH}{\underset{|}{CH}}\right)_{\bar{n}}\!-OH$$

dans laquelle R est un radical hexadécyle et $\bar{n}$ a une valeur statistique moyenne égale à 3 — 4,00 g

— cholestérol — 2,00 g

On réalise le mélange de ces deux produits par fusion à la température de 110°C sous atmosphère d'azote. Puis on ramène la température du mélange fondu à 90°C. On ajoute 20 g d'eau déminéralisée et on homogénéise le mélange obtenu à la température de 90°C. A cette même température, on ajoute 2 g du sel de sodium monohydrate de cholestérol-sulfate et l'on homogénéise le mélange jusqu'à la disparition totale des cristaux lipidiques non associés, que l'on contrôle par examen au microscope optique en lumière polarisée.

On ajoute alors les produits suivants:

| | |
|---|---|
| — parahydroxybenzoate de méthyle (stabilisateur) | 0,30 g |
| — glycérine | 3,00 g |
| — eau déminéralisée | 15,50 g |

A la température de 70°C, on homogénéise le mélange à l'aide d'un ultradisperseur du type "Virtis®" jusqu'à ce que la taille moyenne des vésicules obtenues soit de 0,3 micron ($3.10^{-7}$ m).

2ème phase de préparation:

On ajoute au mélange précédemment obtenu 25 g d'huile de sésame. On soumet le tout à l'action d'un ultradisperseur "Virtis®" jusqu'à ce que les globules d'huile aient un diamètre moyen d'environ 1 micron ($10^{-6}$ m).

On ajoute enfin les produits suivants:

| | |
|---|---|
| — parfum | 0,40 g |
| — acide polyacrylique réticulé vendu sous la dénomination commerciale "CARBOPOL® 940" par la société GOODRICH | 0,40 g |
| — tri-éthanolamine | 0,40 g |
| — eau déminéralisée | 27,00 g |

Cette crème, appliquée en utilisation topique une fois par jour sur des sujets à peau sèche, donne des résultats satisfaisants après 20 jours d'application.

Exemple 2: Creme de soins pour peaux acneiques irritees

1ère phase de préparation:

Dans un ballon rond de 1 litre, on dissout dans 200 ml d'un mélange solvant (chloroforme/méthanol dans le rapport 2/1), les produits suivants:

— lipide amphiphile anionique de formule:

$$C_{15}H_{31}-CHOH-\underset{\displaystyle NH-COR}{\underset{|}{CH}}-COOH,H_2N-CH_2-CHOH-CH_3$$

| | |
|---|---|
| formule dans laquelle R=reste oléyle pour environ 70%, reste hexadécanoyle pour environ 25% et reste tétradécanoyle pour environ 5% (ce lipide appartient à la famille des lipides décrits dans la demande de brevet luxembourgeois n° 85 971) | 4,80 g |
| — cholestérol | 2,80 g |
| — sel de sodium monohydrate du cholestérol-sulfate | 0,40 g |

On évapore le solvant par passage à la pompe à palettes pendant une heure. On met en contact l'association de lipides obtenue avec 20 g d'eau déminéralisée et 3 g de glycérine; on homogénéise le mélange à 90°C.

On ajoute ensuite les produits suivants:

| | |
|---|---|
| — parahydroxybenzoate de méthyle (stabilisateur) | 0,30 g |
| — eau déminéralisée | 32,50 g |

On soumet le tout à l'action d'un ultradisperseur du type "Virtis®" jusqu'à ce que la taille moyenne des vésicules obtenues soit d'environ 0,5 micron ($5.10^{-7}$ m).

2ème phase de préparation:

On ajoute au mélange ci-dessus obtenu 15 g d'huile de silicone volatile. On soumet le tout à l'action d'un ultradisperseur jusqu'à ce que les globules d'huile aient un diamètre moyen inférieur au micron ($10^{-6}$ m).

On ajoute enfin les produits suivants:

| | |
|---|---|
| — parfum | 0,40 g |
| — polyglucose à chaîne linéaire commercialisé par la société "Alban Muller" sous le nom commercial "Amigel® Poudre" | 0,40 g |
| — eau déminéralisée | 20,40 g |

Cette crème, utilisée en application topique deux fois par jour sur des sujets ayant une peau acnéique irritée, diminue l'irritation après une ou deux semaines d'application.

Exemple 3: Concentre pour le traitement de la xerose

Dans un ballon rond de 1 litre, on dissout, dans 200 ml d'un mélange solvant (chloroforme/méthanol dans le rapport 2/1), les produits suivants:

| | |
|---|---|
| — lécithine de soja "Epikuron® E 200" | 12,00 g |
| — sel de sodium monohydrate du cholestérol-sulfate | 4,00 g |
| — DL-α-tocophérol | 1,00 g |

On évapore le solvant à l'aide d'un évaporateur rotatif et on élimine les dernières traces de solvant par passage à la pompe à palettes pendant une heure. On met en contact l'association de lipides obtenue avec 40,00 g d'eau déminéralisée, et on homogénéise le mélange à 40°C.

On ajoute ensuite les produits suivants:

| | |
|---|---|
| — parahydroxybenzoate de méthyle (stabilisateur) | 0,30 g |
| — eau déminéralisée | 42,00 g |
| — parfum | 0,70 g |

On soumet le tout à l'action d'un ultradisperseur du type "Virtis®" jusqu'à ce que la taille moyenne des vésicules obtenues soit inférieure au micron ($10^{-6}$ m).

La dispersion fluide obtenue peut être appliquée sur la peau par projection à partir d'un flacon pompe.

Ce concentré, utilisé en application deux fois par jour, donne des résultats satisfaisants après trois semaines d'application et révèle une excellente tolérance.

Exemple 4: Lait pour le soin des peaux seches

1ère phase de préparation:

Dans un ballon rond de 1 litre, on dissout, dans 200 ml d'un mélange solvant (chloroforme/méthanol dans le rapport 1/1), les produits suivants:

— lipide amphiphile non-ionique de formule

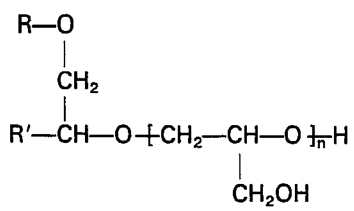

dans laquelle

R est un radical dodécyle
R' est un mélange équimolaire des radicaux tétradécyle et hexadécyle et $\bar{n}$ a une valeur statistique moyenne déterminée par résonance magnétique nucléaire égale à 5,5 — 7,6 g

— sel de potassium du cholestérolsulfate — 0,4 g

On évapore le solvant à l'aide d'un évaporateur rotatif et on élimine les dernières traces de solvant par passage à la pompe à palettes pendant 1 heure.

On met en contact l'association de lipides obtenue avec 20,0 g d'eau déminéralisée et on homogénéise le mélange à 90°C.

On ajoute alors les produits suivants:

| | |
|---|---|
| — parahydroxybenzoate de méthyle (stabilisateur) | 0,3 g |
| — glycérine | 5,0 g |
| — eau déminéralisée | 36,7 g |

A la température de 40°C, on homogénéise le mélange à l'aide d'un ultradisperseur du type "Virtis®" jusqu'à ce que la taille moyenne des sphérules obtenues soit de 0,2 micron ($2.10^{-7}$ m).

2ème phase de préparation:

On ajoute à la dispersion aqueuse ci-dessus 15 g d'huile de sésame.

On soumet le tout à l'action de l'ultradisperseur "Virtis®" jusqu'à ce que les globules d'huile aient un diamètre moyen d'environ 1 micron ($10^{-6}$ m).

On ajoute enfin les substances suivantes:

| | |
|---|---|
| — parfum | 0,4 g |
| — acide polyacrylique réticulé vendu sous la dénomination commerciale "CARBOPOL® 940" par la société GOODRICH | 0,4 g |
| — tri-éthanolamine | 0,4 g |
| — eau déminéralisée | 13,8 g |

Ce lait, utilisé en application topique une fois par jour sur des sujets ayant une peau sèche, donne des résultats satisfaisants après 20 jours d'application.

Exemple 5: Lait pour le soin des peaux irritees

1ère phase de préparation:

Dans un bécher en acier inoxydable, on pèse les produits suivants:

| | |
|---|---|
| — lipide amphiphile non-ionique utilisé dans l'exemple 4 | 3,8 g |
| — sel d'ammonium monohydrate du cholestérol-sulfate | 0,2 g |

On réalise le mélange de ces deux produits par fusion à 95°C. On ajoute 10 g d'eau déminéralisée. On homogénéise le mélange obtenu à 90°C.

On ajoute alors les produits suivants:

| | |
|---|---|
| — parahydroxybenzoate de méthyle (stabilisateur) | 0,3 g |
| — glycérine | 5,0 g |
| — eau déminéralisée | 50,7 g |

On homogénéise le mélange à 40°C à l'aide d'un ultradisperseur du type "Virtis®" jusqu'à ce que la taille moyenne des sphérules obtenues soit de 0,2 micron ($2.10^{-7}$ m).

2ème phase de préparation:

On ajoute à la dispersion aqueuse ci-dessus obtenue 15 g d'huile de sésame. On soumet le tout à l'action de l'ultradisperseur "Virtis®" jusqu'à ce que les globules d'huile aient un diamètre moyen d'environ 1 micron ($10^{-6}$ m).

On ajoute enfin les substances suivantes:

| | |
|---|---|
| — parfum | 0,4 g |
| — acide polyacrylique réticulé vendu sous la dénomination commerciale "CARBOPOL® 940" par la société GOODRICH | 0,4 g |
| — tri-éthanolamine | 0,4 g |
| — eau déminéralisée | 13,00 g |

Ce lait, utilisé en application topique deux fois par jour sur des sujets ayant une peau irritée, diminue l'irritation après une ou deux semaines d'application.

Exemple 6: Creme de soin pour peaux acneiques

Toute la préparation de cette crème a été effectuée à la lumière jaune d'une lampe à vapeur de sodium.

1ère phase de préparation:

Dans un ballon rond de 1 litre, on dissout, dans 200 ml d'un mélange solvant (chloroforme/méthanol dans le rapport 1/1) les produits suivants:

— lipide non-ionique de formule:

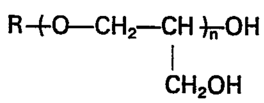

dans laquelle R est un radical hexadécyle et

| | |
|---|---|
| $\bar{n}$ a une valeur statistique moyenne égale à 3 | 3,8 g |
| — cholestérol | 3,8 g |
| — sel de sodium du cholestérol-sulfate | 0,4 g |
| — acide rétinoïque vendu par la société ROCHE sous la dénomination commerciale "Trétinoïne®" | 0,025 g |

On évapore le solvant à l'aide d'un évaporateur rotatif et on élimine les dernières traces de solvant par passage à la pompe à palettes pendant 1 heure.

On met en contact l'association de lipides obtenue avec 20 g d'eau déminéralisée mélangée à 3 g de glycérine. On homogénéise le mélange obtenu à 80°C.

On ajoute alors les produits suivants:

| | |
|---|---|
| — parahydroxybenzoate de méthyle (stabilisateur) | 0,3 g |
| — eau déminéralisée | 38,675 g |

A 60°C, on homogénéise le mélange à l'aide d'un ultradisperseur "Virtis®" jusqu'à ce que la taille moyenne des sphérules obtenues soit d'environ 0,3 micron ($3.10^{-7}$ m).

2ème phase de préparation:

On ajoute alors 15 g de Tricaprocaprylate de glycérol. On soumet le tout à l'action de l'ultradisperseur "Virtis®" de façon que la phase externe de la dispersion d'huile présente des globules d'huile d'un diamètre moyen d'environ 1 micron ($10^{-6}$ m).

On ajoute alors les produits suivants:

| | |
|---|---|
| — parfum | 0,4 g |
| — acide polyacrylique réticulé vendu sous la dénomination commerciale "CARBOPOL® 940" par la société GOODRICH | 0,4 g |
| — tri-éthanolamine | 0,4 g |
| — eau déminéralisée | 13,8 g |

Cette crème, utilisée en application topique deux fois par jour sur des sujets ayant une peau acnéique, permet de constater une amélioration sensible après deux semaines d'application.

Exemple 7: Preparation vesiculaire de corticoides

Dans un bécher en acier inoxydable, on pèse les produits suivants:

| | |
|---|---|
| — lipide amphiphile non-ionique utilisé dans l'exemple 4 | 7,6 g |
| — sel de sodium du cholestérol-sulfate | 0,4 g |
| — 17-valérate de β-méthasone (commercialisé par la société LARKS) | 0,08 g |

On réalise le mélange de ces trois produits par fusion à 90°C.

On rajoute 20 g d'eau déminéralisée et on homogénéise le mélange obtenu à 90°C.

On ajoute alors les produits suivants:

| | |
|---|---|
| — parahydroxybenzoate de méthyle (stabilisateur) | 0,3 g |
| — glycérine | 5,0 g |
| — eau déminéralisée | 52,02 g |

A 40°C, on homogénéise le mélange à l'aide d'un ultradisperseur du type "Virtis®" jusqu'à ce que la taille moyenne des sphérules obtenues soit de 0,2 micron ($2.10^{-7}$ m).

On ajoute enfin les substances suivantes:

| | |
|---|---|
| — acide polyacrylique réticulé vendu sous la dénomination commerciale "CARBOPOL® 940" par la société GOODRICH | 0,4 g |
| — tri-éthanolamine | 0,4 g |
| — eau déminéralisée | 13,8 g |

Cette préparation, utilisée en application topique deux fois par jour sur des sujets présentant une dermatose, permet de constater une amélioration après quelques jours d'application.

Exemple 8: Dispersion aqueuse de vesicules lipidiques

Dans un ballon rond de 1 litre, on dissout, dans 200 ml d'un mélange solvant (chloroforme/méthanol dans le rapport 1/1) les produits suivants:

| | |
|---|---|
| — lipide amphiphile non-ionique utilisé dans l'exemple 4 | 7,6 g |
| — sel de sodium du cholestérol-sulfate | 0,4 g |
| — acétate d'α-tocophérol (vitamine E acétate), produit commercialisé par la société ROCHE | 0,2 g |
| — α-tocophérol (vitamine E), produit commercialisé par la société ROCHE | 0,2 g |
| — palmitate d'ascorbyle (anti-oxydant), produit commercialisé par la société ROCHE | 0,4 g |

On évapore le solvant à l'aide d'un évaporateur rotatif et on élimine les dernières traces de solvant par passage à la pompe à palettes pendant 1 heure. On met en contact l'association de lipides obtenue avec 20 g d'eau déminéralisée et on homogénéise le mélange obtenu à 90°C.

On ajoute alors les produits suivants:

| | |
|---|---|
| — parahydroxybenzoate de méthyle (stabilisateur) | 0,3 g |
| — glycérine | 5,0 g |
| — eau déminéralisée | 51,3 g |

A 40°C, on homogénéise le mélange à l'aide d'un ultradisperseur "Virtis®" jusqu'à ce que la taille moyenne des vésicules obtenues soit de 0,2 micron ($2.10^{-7}$ m).

On ajoute enfin les substances suivantes:

| | |
|---|---|
| — acide polyacrylique réticulé vendu sous la dénomination commerciale "CARBOPOL® 940" par la société GOODRICH | 0,4 g |
| — tri-éthanolamine | 0,4 g |
| — eau déminéralisée | 13,8 g |

Cette dispersion, utilisée en application topique une fois par jour sur des sujets ayant une peau présentant certains signes de vieillissement, donne des résultats satisfaisants après quatre semaines d'application.

**Revendications**

1. Procédé pour faciliter la formation de sphérules lipidiques en dispersion dans un milieu aqueux D, et pour améliorer simultanément la stabilité et le taux d'encapsulation desdites sphérules lipidiques, celles-ci étant constituées de feuillets lipidiques sensiblement concentriques encapsulant une phase aqueuse E, le(s) lipide(s) constitutif(s) des feuillets étant des amphiphiles ioniques ou non-ioniques, caractérisé par le fait qu'avant la formation desdites sphérules, on ajoute au(x) lipide(s) destiné(s) à constituer les feuillets des sphérules, au moins un cholestérol-sulfate de cation ammonium, alcalin ou alcalino-terreux, à raison de 1 à 50% en poids par rapport au poids total de la phase lipidique.

2. Procédé selon la revendication 1, caractérisé par le fait qu'on utilise un cholestérol-sulfate d'ammonium substitué ou non, de sodium ou de potassium.

3. Procédé selon l'une des revendication 1 ou 2, caractérisé par le fait que, pour obtenir une dispersion de sphérules dans la phase D, on dissout les lipides dans un solvant volatil, on forme un film mince de lipides sur les parois d'un flacon par évaporation du solvant, on introduit dans ledit flacon la phase aqueuse E à encapsuler et on agite le mélange mécaniquement jusqu'à l'obtention de la dispersion de sphérules à la taille désirée, les phases aqueuses D et E étant nécessairement identiques.

4. Procédé selon l'une des revendications 1 ou 2, caractérisé par le fait que, pour obtenir une dispersion de sphérules dans la phase D, on forme une phase lamellaire plane par introduction de la phase aqueuse à encapsuler E dans les lipides liquides, à une température légèrement supérieure à la température de fusion des lipides, on ajoute ensuite à la phase lamellaire obtenue, une phase aqueuse de dispersion D, qui peut être identique ou non à la phase aqueuse E, on agite énergiquement pour obtenir le passage de la phase lamellaire plane à une dispersion, dans la phase aqueuse D, de sphérules lipidiques encapsulant la phase aqueuse E.

5. Procédé selon l'une des revendications 1 à 4, caractérisé par le fait que, comme lipides destinés à constituer les feuillets des sphérules, on choisit au moins un amphiphile d'origine naturelle ou synthétique, ionique ou non-ionique, comportant par molécule une ou plusieurs longue(s) chaîne(s) hydrocarbonée(s) comprenant 8 à 30 atomes de carbone et un ou plusieurs groupement(s) hydrophile(s) pris parmi les groupes hydroxyle, éthéroxyde, carboxyle, phosphate et amine.

6. Procédé selon la revendication 5, caractérisé par le fait que, comme amphiphile ionique, on choisit au moins un produit pris dans le groupe formé par les phospholipides naturels, tels que la lécithine d'oeuf ou de soja et la sphingomyéline, les phospholipides de synthèse, tels que la dipalmitoyl-phosphatidylcholine ou la lécithine hydrogénée, les composés amphotères comportant deux chaînes lipophiles ou une association de deux ions organiques à longue chaîne de signes opposés et les composés anioniques.

7. Procédé selon la revendication 5, caractérisé par le fait que, comme amphiphile non-ionique, on choisit au moins un composé pris dans le groupe formé par:

— les éthers de polyglycérol, linéaires ou ramifiés, de formules respectives:

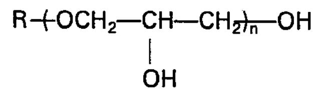

et

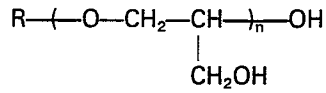

$\bar{n}$ représentant une valeur statistique moyenne comprise entre 1 et 6, R représentant une chaîne aliphatique linéaire ou ramifiée, saturée ou insaturée, comprenant 12 à 30 atomes de carbone, des radicaux hydrocarbonés des alcools de lanoline ou les restes hydroxy-2 alkyle des α-diols à longue chaîne;
— les éthers de polyglycérol, linéaires ou ramifiés, comportant deux chaînes grasses;
— les alcools gras polyoxyéthylénés ou les stérols polyoxyéthylénés;
— les éthers de polyols;
— les esters de polyols, oxyéthylénés ou non;
— les glycolipides d'origine naturelle ou synthétique;
— les hydroxyamides représentés par la formule:

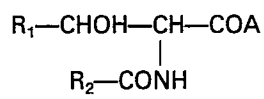

formule dans laquelle:
— $R_1$ désigne un radical alcoyle ou alcényle en $C_7$—$C_{21}$;
— $R_2$ désigne un radical hydrocarboné, saturé ou insaturé en $C_7$—$C_{31}$;
— COA désigne un groupement choisi parmi les deux groupements suivants:
— un reste

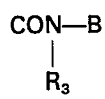

B étant un radical dérivé d'amines primaires ou secondaires, mono- ou polyhydroxylées et $R_3$ désignant un atome d'hydrogène ou un radical méthyle, éthyle ou hydroxyéthyle;
— COOZ, Z représentant le reste d'un polyol en $C_3$—$C_7$.

8. Procédé selon l'une des revendications 1 à 7, caractérisé par le fait que l'on ajoute aux amphiphiles destinés à former les sphérules, au moins un additif pris dans le groupe formé par les alcools et diols à longue chaîne, les stérols, les amines à longue chaîne, les hydroxyalkylamines, les amines grasses polyoxyéthylénées, les esters d'amino-alcools à longue chaîne, et leurs sels, les esters phosphoriques d'alcools gras, les alkyl-sulfates et les dérivés ioniques de stérols autres que les cholestérol-sulfates.

9. Procédé selon l'une des revendications 1 à 8, caractérisé par le fait que l'on utilise, pour constituer la dispersion de sphérules, de 0,5 à 25% en poids d'amphiphile(s) par rapport au poids total de la dispersion de sphérules à obtenir.

10. Procédé selon l'une des revendications 1 à 9, caractérisé par le fait que l'on ajoute aux amphiphiles destinés à former les sphérules au moins une substance liposoluble active comme, par exemple, les agents kératolitiques, les agents anti-inflammatoires et les agents anti-oxydants.

11. Procédé selon l'une des revendications 1 à 10, caractérisé par le fait que la phase aqueuse E encapsulée dans les sphérules est une solution aqueuse de substance(s) active(s), de préférence isoosmotique par rapport à la phase D qui entoure les sphérules.

12. Procédé selon la revendication 11, caractérisé par le fait que les phases aqueuses D et E sont identiques.

13. Procédé selon l'une des revendications 11 ou 12, conduisant à une composition utilisable en cosmétique, caractérisé par le fait que l'on introduit dans la phase aqueuse E au moins un produit pris dans

le groupe formé par les humectants, les agents de brunissage articiel, les agents anti-solaires hydrosolubles, les agents anti-perspirants, les déodorants, les astringents, les produits rafraîchissants, les produits toniques, les produits cicatrisants, les produit kératolytiques, les produits dépilatoires, les eaux parfumées, les colorants hydrosolubles, les agents anti-pelliculaires, les agents anti-séborrhéiques, les oxydants, les réducteurs et les extraits de tissus animaux ou végétaux.

14. Procédé selon l'une des revendications 11 ou 12, conduisant à une composition utilisable en pharmacie, caractérisé par le fait que l'on introduit dans la phase aqueuse E, au moins un produit pris dans le groupe formé par les vitamines, les hormones, les enzymes, les vaccins, les anti-inflammatoires, les antibiotiques et les bactéricides.

15. Procédé selon l'une des revendications 1 à 14, caractérisé par le fait que l'on mélange la dispersion de sphérules avec au moins une phase liquide non miscible à l'eau, destinée à être dispersée dans la phase aqueuse D.

16. Procédé selon la revendication 15, caractérisé par le fait que l'on introduit une quantité de phase liquide non miscible à l'eau comprise entre 2 et 70% en poids par rapport au poids total de la composition, la proportion pondérale relative de lipide(s) amphiphile(s) constitutif(s) de sphérules par rapport à la phase liquide non miscible à l'eau dispersée étant comprise entre 0,02/1 et 10/1.

17. Procédé selon l'une des revendications 15 ou 16, caractérisé par le fait que l'on choisit la phase liquide non miscible à l'eau dispersée dans la phase aqueuse D dans le groupe constitué par les huiles telles que les esters d'acides gras et de polyols, et les esters d'acides gras et d'alcools ramifiés de formule $R_4$—$COOR_5$, formule dans laquelle $R_4$ représente le reste d'un acide gras supérieur comportant de 7 à 19 atomes de carbone et $R_5$ représente une chaîne hydrocarbonée ramifiée contenant de 3 à 20 atomes de carbone, les hydrocarbures tels que l'hexadécane, l'huile de paraffine, le perhydrosqualène; les carbures halogénés tels que le perfluorodécahydronaphtalène; la perfluorotributylamine; les polysiloxanes; les esters d'acides organiques; les éthers et polyéthers.

18. Procédé selon l'une des revendications 1 à 17, caractérisé par le fait que l'on introduit dans la phase aqueuse D au moins un adjuvant pris dans le groupe formé par les opacifiants, les gélifiants, les arômes, les parfums, les filtres anti-solaires et les colorants.

19. Dispersion de sphérules lipidiques dans un milieu aqueux D, lesdites sphérules étant constituées de feuillets lipidiques sensiblement concentriques encapsulant une phase aqueuse E, les lipides constitutifs des feuillets étant des amphiphiles ioniques ou non-ioniques, caractérisée par le fait qu'elle est obtenue par le procédé selon l'une des revendications 1 à 18.

**Patentansprüche**

1. Verfahren zur leichteren Herstellung einer Dispersion von Lipidkügelchen in einem wäßrigen Milieu D und zur gleichzeitigen Verbesserung der Stabilität und des Einkapselungsgrades der Lipidkügelchen, wobei die Lipidkügelchen aus im wesentlichen konzentrischen Lipidschalen bestehen, die eine wäßrige Phase E einkapseln und wobei das (die) Lipid(e) der Schalen ionische oder nicht-ionische Amphiphile sind, dadurch gekennzeichnet, daß man vor der Bildung der Lipidkügelchen wenigstens ein Ammonium-, Alkalimetall- oder Erdalkalimetall-Cholesterinsulfat in einer Menge von 1 bis 50 Gew.-%, bezogen auf das Gesamtgewicht der Lipidphase, zu dem (den) Lipid(en) gibt, das (die) für die Bildung der Schalen der Kügelchen bestimmt ist (sind).

2. Verfahren nach Anspruch 1, dadurch gekennzeichnet, daß man substituiertes oder unsubstituiertes Ammonium-, Natrium- oder Kalium-Cholesterinsulfat verwendet.

3. Verfahren nach einem der Ansprüche 1 oder 2, dadurch gekennzeichnet, daß man zur Herstellung einer Dispersion von Kügelchen in der Phase D die Lipide in einem flüchtigen Lösungsmittel löst, durch Verdampfen des Lösungsmittels einen dünnen Lipidfilm auf der Wand eines Behälters bildet, die einzukapselnde wäßrige Phase E in den Behälter gibt und auf mechanische Weise das Gemisch so lange rührt, bis man die Dispersion von Kügelchen in der gewünschten Größe erhält, wobei die wäßrigen Phasen D und E notwendigerweise gleich sind.

4. Verfahren nach einem der Ansprüche 1 oder 2, dadurch gekennzeichnet, daß man zur Herstellung einer Dispersion von Kügelchen in der Phase D eine plane, lamellare Phase durch Einbringen der einzukapselnden wäßrigen Phase E in das (die) flüssige(n) Lipid(en) bildet, anschließend eine wäßrige Dispersionsphase D, die gleich oder verschieden von der wäßrigen Phase E sein kann, zur erhaltenen lamellaren Phase gibt und heftig bewegt, damit die plane lamellare Phase in eine Dispersion der Lipidkügelchen, die die wäßrige Phse E einkapseln, in der wäßrigen Phase D übergeht.

5. Verfahren nach einem der Ansprüche 1 bis 4, dadurch gekennzeichnet, daß man als Lipide, die für die Bildung der Schalen der Kügelchen bestimmt sind, mindestens ein ionisches oder nicht-ionisches Amphiphil natürlicher oder synthetischer Herkunft verwendet, das pro Molekül eine oder mehrere lange Kohlenwasserstoffketten mit 8 bis 30 Kohlenstoffatomen und eine oder mehrere hydrophile Gruppen, ausgewählt unter Hydroxyl-, Etheroxid-, Carboxyl-, Phosphat- und Amingruppen, besitzt.

6. Verfahren nach Anspruch 5, dadurch gekennzeichnet, daß man als ionisches Amphiphil mindestens ein Produkt verwendet, das ausgewählt ist unter natürlichen Phospholipiden, wie Ei- oder Sojalecithin und Sphingomyelin, synthetischen Phospholipiden, wie Dipalmitoyl-Phosphatidylcholin oder hydriertes

Lecithin, amphoteren Verbindungen mit zwei lipophilen Ketten oder mit einer Assoziation von zwei organischen, langkettigen Ionen mit entgegengesetztem Vorzeichen, und anionischen Verbindungen.

7. Verfahren nach Anspruch 5, dadurch gekennzeichnet, daß man als nicht-ionisches Amphiphil mindestens eine Verbindung verwendet, die ausgewählt ist unter

— geradkettigen oder verzweigten Polyglycerinethern der Formeln

$$R\text{—}(\text{—}OCH_2\text{—}CH\text{—}CH_2\text{—})_n\text{—}OH$$
$$|$$
$$OH$$

und

$$R\text{—}(\text{—}O\text{—}CH_2\text{—}CH\text{—})_n\text{—}OH$$
$$|$$
$$CH_2OH$$

worin

n einen statistischen Mittelwert von 1 bis 6 bedeutet,

R eine geradkettige oder verzweigte, gesättigte oder ungesättigte aliphatische Kette mit 12 bis 30 Kohlenstoffatomen, Kohlenwasserstoffreste von Lanolinalkoholen oder 2-Hydroxyalkylreste von -Diolen mit langer Kette bedeutet;

— geradkettigen oder verzweigten Polyglycerinethern mit 2 Fettketten;

— polyoxyethylenierten Fettalkoholen oder polyoxyethylenierten Sterinen;

— Polyolethern;

— gegebenenfalls oxyethylenierten Polyolestern;

— Glykolipiden natürlicher oder synthetischer Herkunft und

— Hydroxyamiden der allgemeinen Formel

$$R_1\text{—}CHOH\text{—}CH\text{—}COA$$
$$|$$
$$R_2\text{—}CONH$$

worin

$R_1$ einen $C_7$—$C_{21}$-Alkyl- oder -Alkenylrest bedeutet;

$R_2$ einen gesättigten oder ungesättigten $C_7$—$C_{31}$-Kohlenwasserstoffrest bedeutet;

COA eine Gruppe bedeutet, die ausgewählt ist unter den beiden folgenden Gruppen:

einem Rest der Formel

$$CON\text{—}B$$
$$|$$
$$R_3$$

worin B einen von einem primären oder sekundären, mono- oder polyhydroxylierten Amin abgeleiteten Rest bedeutet und $R_3$ ein Wasserstoffatom oder einen Methyl-, Ethyl- oder Hydroxyethylrest bedeutet;

einem Rest der Formel COOZ,

worin Z den Rest eines $C_3$—$C_7$-Polyols bedeutet.

8. Verfahren nach einem der Ansprüche 1 bis 7, dadurch gekennzeichnet, daß man zu den für die Bildung der Kügelchen bestimmten Amphiphilen mindestens ein Additiv gibt, ausgewählt unter langkettigen Alkoholen und Diolen, Sterinen, langkettigen Aminen, Hydroxyalkylaminen, polyoxyethylenierten Fettaminen, Estern von Aminoalkoholen mit langer Kette und den Salzen davon, Phosphorsäureestern von Fettalkoholen, Alkylsulfaten und anderen ionischen Sterinderivaten, die von Cholesterinsulfaten verschieden sind.

9. Verfahren nach einem der Ansprüche 1 bis 8, dadurch gekennzeichnet, daß man zur Bildung der Kügelchendispersion 0,5 bis 25 Gew.-% Amphiphil(e), bezogen auf das Gesamtgewicht der herzustellenden Kügelchendispersion, verwendet.

10. Verfahren nach einem der Ansprüche 1 bis 9, dadurch gekennzeichnet, daß man zu den zur Bildung der Kügelchen bestimmten Amphiphilen mindestens einen fettlöslichen Wirkstoff gibt, beispielsweise ein keratolytisches Mittel, ein antiinflammatorisches Mittel oder ein Antioxidans.

11. Verfahren nach einem der Ansprüche 1 bis 10, dadurch gekennzeichnet, daß die in den Kügelchen eingekapselte wäßrige Phase E eine wäßrige Lösung des (der) Wirkstoffes (Wirkstoffe) ist, die vorzugsweise isoosmotisch mit der Phase D ist, welche die Kügelchen umgibt.

12. Verfahren nach Anspruch 11, dadurch gekennzeichnet, daß die wäßrigen Phasen D und E identisch sind.

13. Verfahren nach einem der Ansprüche 11 oder 12 zur Herstellung eines kosmetischen Mittels, dadurch gekennzeichnet, daß man in die wäßrige Phase E mindestens ein Produkt einbringt, das

ausgewählt ist unter Befeuchtungsmitteln, künstlichen Bräunungsmitteln, wasserlöslichen Sonnenschutzmitteln, Antiperspirantien, Deodorantien, adstringierenden Mitteln, erfrischenden Produkten, Stärkungsmitteln, narbenverheilenden Produkten, keratolytischen Produkten, depilatorischen Produkten, Parfümwässern, wasserlöslichen Farbstoffen, Antischuppen-Mitteln, Antiseborrhoe-Mitteln, Oxidationsmitteln, Reduktionsmitteln und Extrakten tierischer oder pflanzlicher Gewebe.

14. Verfahren nach einem der Ansprüche 11 oder 12 zur Herstellung eines pharmazeutischen Mittels, dadurch gekennzeichnet, daß man in die wäßrige Phase E mindestens ein Produkt einbringt, das ausgewählt ist unter Vitaminen, Hormonen, Enzymen, Vakzinen, antiinflammatorischen Mitteln, Antibiotika und Bakteriziden.

15. Verfahren nach einem der Ansprüche 1 bis 14, dadurch gekennzeichnet, daß man die Kügelchendispersion mit wenigstens einer flüssigen, mit Wasser nicht mischbaren Phase vermischt, die dazu bestimmt ist, in der wäßrigen Phase D dispergiert zu werden.

16. Verfahren nach Anspruch 15, dadurch gekennzeichnet, daß man die flüssige, mit Wasser nicht mischbare Phase in einer Menge von 2 bis 70 Gew.-%, bezogen auf das Gesamtgewicht des Mittels, einbringt, wobei das Gewichtsverhältnis von dem (den) amphiphilen Lipid(en), das (die) Kügelchen bildet, zu der flüssigen, mit Wasser nicht mischbaren, dispergierten Phase 0,02/1 bis 10/1 beträgt.

17. Verfahren nach einem der Ansprüche 15 oder 16, dadurch gekennzeichnet, daß die flüssige, mit Wasser nicht mischbare, in der wäßrigen Phase D dispergierte Phase ausgewählt ist unter Ölen, wie Estern von Fettsäuren und Polyolen und Estern von Fettsäuren und verzweigten Alkoholen der Formel $R_4$—$COOR_5$, worin $R_4$ einen Fettsäurerest mit 7 bis 19 Kohlenstoffatomen bedeutet und $R_5$ eine verzweigte Kohlenwasserstoffkette mit 3 bis 20 Kohlenstoffatomen bedeutet, Kohlenwasserstoffen, wie Hexadekan, Paraffinölen, Perhydrosqualen; halogenierten Kohlenstoffverbindungen, wie Perfluordekahydronaphthalin; Perfluortributylamin; Polysiloxanen; Estern organischer Säuren; Ethern und Polyethern.

18. Verfahren nach einem der Ansprüche 1 bis 17, dadurch gekennzeichnet, daß man in die wäßrige Phase D wenigstens ein Adjuvans einbringt, ausgewählt unter opakmachenden Mitteln, gebildenden Mitteln, Aromastoffen, Parfums, Sonnenfiltern und Farbstoffen.

19. Dispersion von Lipidkügelchen in einem wäßrigen Milieu D, wobei die Kügelchen aus im wesentlichen konzentrischen Lipidschalen bestehen, die eine wäßrige Phase E einkapseln und wobei die die Schalen bildenden Lipide ionische oder nicht-ionische Amphiphile sind, dadurch gekennzeichnet, daß sie gemäß einem Verfahren nach einem der Ansprüche 1 bis 18 erhältlich ist.

## Claims

1. Process for facilitating the formation of lipid spherules in dispersion in an aqueous medium D and for simultaneously improving the stability and the degree of encapsulation of the said lipid spherules, the latter consisting of substantially concentric lipid leaflets encapsulating an aqueous phase E, the lipid(s) of which the leaflets consist being ionic or nonionic amphiphiles, characterized in that before the formation of the said spherules, at least one ammonium, alkali metal or alkaline-earth metal cation cholesterol sulphate is added to the lipid(s) intended to form the leaflets of the spherules, in a proportion of 1 to 50% by weight relative to the total weight of the lipid phase.

2. Process according to Claim 1, characterized in that a substituted or unsubstituted ammonium, sodium or potassium cholesterol sulphate is employed.

3. Process according to either of Claims 1 and 2, characterized in that, to obtain a dispersion of spherules in the phase D, the lipids are dissolved in a volatile solvent, a thin film of lipids is formed on the walls of a flask by evaporating the solvent, the aqueous phase E to be encapsulated is introduced into the said flask and the mixture is agitated mechanically until the dispersion of spherules of the desired size is obtained, the aqueous phases D and E being necessarily identical.

4. Process according to either of Claims 1 and 2, characterized in that, to obtain a dispersion of spherules in the phase D, a planar lamellar phase is formed by introducing the aqueous phase to be encapsulated E into the liquid lipids, at a temperature slightly higher than the melting temperature of the lipids, an aqueous dispersion phase D, which may be identical or otherwise to the aqueous phase E is then added to the lamellar phase obtained, and energetic stirring is performed to obtain the transition from the planar lamellar phase to a dispersion, in the aqueous phase D, of lipid spherules encapsulating the aqueous phase E.

5. Process according to one of Claims 1 to 4, characterized in that the chosen lipids intended to form the leaflets of the spherules are at least one ionic or nonionic amphiphile of natural or synthetic origin containing, per molecule, one or more long hydrocarbon chain(s) containing 8 to 30 carbon atoms and one or more hydrophilic group(s) taken from hydroxyl, ether, carboxyl, phosphate and amine groups.

6. Process according to Claim 5, characterized in that the chosen ionic amphiphile is at least one product taken from the group consisting of natural phospholipids, such as egg or soya lecithin or sphingomyelin, synthetic phospholipids, such as dipalmitoylphosphatidylcholine or hydrogenated lecithin, amphoteric compounds containing two lipophilic chains or a combination of two long-chain organic ions of opposite signs, and anionic compounds.

7. Process according to Claim 5, characterized in that the chosen nonionic amphiphile is at least one compound taken from the group consisting of:

— linear or branched polyglycerol ethers, of respective formulae:

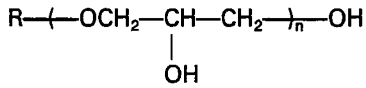

and

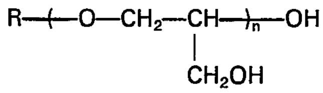

n denoting a mean statistical value of between 1 and 6, R denoting a saturated or unsaturated, linear or branched aliphatic chain containing 12 to 30 carbon atoms, hydrocarbon radicals of lanolin alcohols or 2-hydroxyalkyl residues of long-chain α-diols;

— linear or branched polyglycerol ethers containing two fatty chains;
— polyoxyethylenated fatty alcohols or polyoxyethylenated sterols;
— polyol ethers;
— polyol esters, oxyethylenated or otherwise;
— glycolipids of natural or synthetic origin;
— hydroxyamides denoted by the formula:

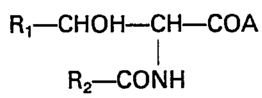

in which formula:

— $R_1$ denotes a $C_7$—$C_{21}$ alkyl or alkenyl radical;
— $R_2$ denotes a $C_7$—$C_{31}$ saturated or unsaturated hydrocarbon radical;
— COA denotes a group chosen from the following two groups;
— a 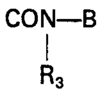 residue

B being a radical derived from mono- or polyhydroxylated primary or secondary amines and $R_3$ denoting a hydrogen atom or a methyl, ethyl or hydroxyethyl radical;

— COOZ, Z denoting the residue of a $C_3$—$C_7$ polyol.

8. Process according to one of Claims 1 to 7, characterized in that to the amphiphiles intended to form the spherules there is added at least one additive taken from the group consisting of long-chain alcohols and diols, sterols, long-chain amines, hydroxyalkylamines, polyoxyethylenated fatty amines, esters of long-chain aminoalcohols and salts thereof, phosphoric esters of fatty alcohols, alkyl sulphates and ionic derivatives of sterols other than cholesterol sulphates.

9. Process according to one of Claims 1 to 8, characterized in that, to form the spherule dispersion, from 0.5 to 25% by weight of amphiphile(s) is (are) employed, relative to the total weight of the spherule dispersion to be obtained.

10. Process according to one of Claims 1 to 9, characterized in that to the amphiphiles intended to form the spherules there is added at least one active liposoluble substance such as, for example, keratolytic agents, antiinflammatory agents and antioxidant agents.

11. Process according to one of Claims 1 to 10, characterized in that the aqueous phase E encapsulated in the spherules is an aqueous solution of active substance(s), preferably isoosmotic relative to the phase D which surrounds the spherules.

12. Process according to Claim 11, characterized in that the aqueous phases D and E are identical.

13. Process according to either of Claims 11 and 12, resulting in a composition capable of being employed in cosmetics, characterized in that into the aqueous phase E there is introduced at least one product taken from the group consisting of humectants, artificial tanning agents, water-soluble sunscreen agents, antiperspirant agents, deodorants, astringents, freshening products, tonic products, cicatrizing products, keratolytic products, depilatory products, perfumed waters, water-soluble dyes, antidandruff agents, antiseborrhoeic agents, oxidizing agents, reducing agents and animal or plant tissue extracts.

14. Process according to either of Claims 11 and 12, resulting in a composition capable of being employed in pharmacy, characterized in that into the aqueous phase E there is introduced at least one product taken from the group consisting of vitamins, hormones, enzymes, vaccines, antiinflammatories, antibiotics and bactericides.

15. Process according to one of Claims 1 to 14, characterized in that the spherule dispersion is mixed with at least one water-immiscible liquid phase intended to be dispersed in the aqueous phase D.

16. Process according to Claim 15, characterized in that a quantity of water-immiscible liquid phase of between 2 and 70% by weight relative to the total weight of the composition is introduced, the relative weight proportion of amphiphile lipid(s) of which the spherules consist relative to the dispersed water-immiscible liquid phase being between 0.02/1 and 10/1.

17. Process according to either of Claims 15 and 16, characterized in that the water-immiscible liquid phase dispersed in the aqueous phase D is chosen from the group consisting of oils such as esters of fatty acids and of polyols, and the esters of fatty acids and of branched alcohols of formula $R_4$—$COOR_5$, in which formula $R_4$ denotes the residue of a higher fatty acid containing from 7 to 19 carbon atoms and $R_5$ denotes a branched hydrocarbon chain containing from 3 to 20 carbon atoms, hydrocarbons such as hexadecane, paraffin oil, perhydrosqualene; halogenated carbides such as perfluorodecahydronaphthalene; perfluorotributylamine; polysiloxanes; esters of organic acids; and ethers and polyethers.

18. Process according to one of Claims 1 to 17, characterized in that into the aqueous phase D there is introduced at least one adjuvant taken from the group consisting of opacifiers, gelling agents, aromas, perfumes, sunscreen filters and dyes.

19. Dispersion of lipid spherules in an aqueous medium D, the said spherules consisting of substantially concentric lipid leaflets encapsulating an aqueous phase E, the lipids of which the leaflets consist being ionic or nonionic amphiphiles, which is characterized in that it is obtained by the process according to one of Claims 1 to 18.